# EUROPEAN PATENT APPLICATION

(11) **EP 4 600 344 A1**
(43) Date of publication of application: **13.08.2025**
(21) Application number: 23874801.6
(22) Date of filing: 02.10.2023
(51) Int. Cl.: C12N 1/21, C12N 1/20, C12N 9/04, C12N 15/53, C12P 7/42

(54) **MICROBIAL STRAIN, PROTEIN, AND METHOD FOR PRODUCING GALLIC ACID USING MICROBIAL STRAIN OR PROTEIN**

(30) Priority: 03.10.2022 JP 2022159637
(71) Applicant: University of Tsukuba, Ibaraki 305-8577 (JP)
(72) Inventor: TAKAYA Naoki, Tsukuba-shi, Ibaraki 305-8577 (JP); NUKUI Noriyuki, Tsukuba-shi, Ibaraki 305-8577 (JP); KATSUKI Nozomi, Tsukuba-shi, Ibaraki 305-8577 (JP); MASUO Shunsuke, Tsukuba-shi, Ibaraki 305-8577 (JP)
(74) Representative: Godemeyer Blum Lenze Patentanwälte Partnerschaft mbB - werkpatent
(86) International application number: PCT/JP2023/035854
(87) International publication number: WO 2024/075674

(57) **Abstract**

An object of the present technology is to provide a protein having protocatechuic acid 5-oxidization activity which can produce gallic acid from protocatechuic acid efficiently and a microbial strain which expresses the protein. Using a protocatechuic acid 5-hydroxylase derived from a microorganism of the genus *Comamonas* or a protein having an identity of 70% or more with the amino acid sequence of the protocatechuic acid 5-hydroxylase, gallic acid can be produced efficiently from protocatechuic acid. Moreover, through fermentation using a microbial strain obtained by introducing a gene encoding the enzyme, gallic acid can be produced from protocatechuic acid.

### Sequence Listing

<110> University of Tsukuba
<120> Microbial strain, protein, method for producing gallic acid using microbial strain or protein
<130> FPCT1388
<160> 42
<210> SEQ ID NO:1
   <211> Length 1209
   <212> Type DNA
   <213> Organism Hylemonella gracilis
<400> Sequence 1 
ATGTCTTATCCCACAAGAACTCAGGTCGCTATCATCGGTGCCGGACCTAGTGGCCTCA

TGCTGGGCGCTCTGCTGCACAAAGCGGGGATTGACACCGTAGTCCTCGAACGGCAGTC

TGGCGATTATGTGCTTGGACGCATTAGAGCCGGCGTTTTGGAACAAGTGACGATGGAC

CTTATGGACGAGATAGGAGTAGGGGCCAGAATGCACCAGGAAGGTCTGGTGCATGGAG

GCTTCGATATGCTCTTTCAGGGGCAACGCCATAGGATTGACATGAATCGGCTGACTGG

AGGTCAGAATGTGATGGTTTATGGGCAGACAGAGGTGACAAGAGACTTGATGGATGCA

AGGCAGCAAGCGGGACTGACCACCATCTACGAAGCCGCTAATGTGGCCATACACGACT

TTGGCAGCGGGAAACCTCGAGTGACTTATGAGAAAGACGGCAAAACCCACGAACTGCA

GTGCGACTTCATTGCAGGCTGCGACGGCTTTCACGGCGTCTGTCGGGATACCGTGAAG

AAGTCCGCCAGTGCCAGTGCAATCCGCGAGTACGAAAAGGTGTACAACTTCGGGTGGC

TGGGTTTGCTGTCCGATACCCCTCCGGTCCACCACGAACTCATCTACGTAAACAGCGA

GAGAGGCTTCGCCCTGTGTTCCCAGAGGTCTGCGACGAGGAGCCGGTATTACCTCCAG

GTGCCACTGACAGATAAGGTCGAACAGTGGTCAGATCAGGCCTTTTGGGATGAGCTCA

AACTGCGCCTTGACCCACAAGCCAGAGAGCATCTGGTTACCGGACCCAGCATAGAGAA

GAGCATCGCACCCCTCAGGTCCTTCGTGACAGAGCCACTGCGGTTTGGGCGACTGTTC
 
CTTGCTGGCGATGCTGGGCACATCGTTCCTCCGACTGGCGCAAAGGGATTGAACCTGG

CCGCCACCGACGTCAAGTATCTCAGCGCTGCACTGACCGAGTTCTACCAGGGAAAGTC

CGAGGCTGGGATCAACCACTACTCAGAACGCTGCCTGAAGAGGATTTGGCGAGCTGAG

AGGTTCTCATGGTGGTTTACGTCCCTGATGCATCGGTTCCCCGAAAATGGCGAGATTG

GGCAGAAATTGCAAGAGGCCGAACTGGACTACATCGTCCATTCCGAGACTGGTGCCAG

GTCTGTGGCGGAAAACTACGTGGGACTGCCCCTCGACTTCGGTGCCTAA
<210> SEQ ID NO:2
   <211> Length 48
   <212> Type DNA
   <213> Organism Hylemonella gracilis
<400> Sequence 2
   ATCACCACAGCCAGGATCCGAATTCAAGCTATCCCACAAGAACTCAGG
<210> SEQ ID NO:3
   <211> Length 43
   <212> Type DNA
   <213> Organism Hylemonella gracilis
<400> Sequence 3
   TTAAGCATTATGCGGCCGCAAGCTTAGGCACCGAAGTCGAGGG
<210> SEQ ID NO:4
   <211> Length 1176
   <212> Type DNA
   <213> Organism Hydrogenophaga sp.
<400> Sequence 4 
ATGCGCACCCAGGTTGGTATCATCGGCGCCGGCCCGGCCGGATTGATGCTCGCGCACA
TGCTTCACCTTGAGGGCATTGAATCTGTAATTATCGAACGTAGCAAGCGCGAGCATGT
ACAAAATCGTTTGCGCGCCGGCGTTCTGGAACAGGGCACGGTAGAAATGATGCGTGAA
CTCGGCTTGGGAGAACGCATCGGACAGCTGGGATTGGAGCAACATCGTATCGACTTTC
GTTTTGGAGGAGAATCCCATCCCATTGATTTCCACGAAGCTACCGGCGGACGGTCGGC
TTGGGTATATCCCCAACACGAAGTGGTCAAAGATCTGATGGCTGCACGTACCGCTGCC
GGTGCCCAGATGCTCTTCGAAACGCCTGTTACTGCTATTGAAGGAATTGACGGCACCC
GCCCAGTAGTTCGTTTTGAAAAGGACGGAGTCGCGGGAGAACTGCACTGTGATTTCGT
TGTTGGTTGTGACGGTTTTCGTGGTATTTCACGTCGTGCTATCCCAGCCCGTGAAGGA
CAAGTGCACGATCGGATCTATCCGTTTGGTTGGCTGGGTATCCTCGCCGAGGCTCCAC
CCGCGACTAATGAAGTAACCTGGGGCTGTCACGAGGACGGCTTTGCTATGATGTCCAT
TCGGACGCCTAGCGTAACCCGTTTGTATCTGCAGTGCGAACCTGATGAAAACCCGGAC
CATTGGTCCGACGACCGTATTTGGTCGGCACTGCATAAGCGGTTGGACGTGCCTGGAT
TGCCACCAATCAACGAAGGTCGCATTACCCAGAAAGGCGTAACGGCCATGCGTTCTTT
TTTGTTTGAGCCCATGCAACACGGTCGTCTGTTTCTGGCCGGTGACGCCGCTCACATC
GTCCCTCCGACCGGCGCCAAGGGACTGAACTCTGCGATGGCAGACGTAAAAGTCCTCG
CCCGCGCGTTGGAGCAACACTACCAACGTGGATCCCAGGACCTTTTGGCTCGTTATTC
AAAGACTTGTCTGAAGCGCATGTGGTTGGTTCAACGGTTCTCGGCCGGACTCTGCACG
ATGGTACATAGCTTCGCAGGCGATTCTTCTTTCGTTCGTCGCCTTCAGCGCGCTGACT
TGGATTACATGACCGGCACGACCGCGGGCCGCTTGCAGTTCTCAGAGAATTTCACGGG
CTTGCCCATCGACTAA
<210> SEQ ID NO:5
   <211> Length 43
   <212> Type DNA
   <213> Organism Hydrogenophaga sp.
<400> Sequence 5
   ATCACCACAGCCAGGATCCGAATTCGCGGACCCAGGTCGGCAT
<210> SEQ ID NO:6
   <211> Length 61
   <212> Type DNA
   <213> Organism Hydrogenophaga sp.
<400> Sequence 6
   TTAAGCATTATGCGGCCGCAAGCTTATTCAATCGGAAGTCCAGTGAAATTCTCTGAAA ACG
<210> SEQ ID NO:7
   <211> Length 1176
   <212> Type DNA
   <213> Organism Polaromonas sp.
<400> Sequence 7 
ATGCGGACCCAGGTCGGCATCGTGGGTGCGGGTCCTGCCGGATTGATGTTGTCACACA

TGCTCCACCTTGAAGGTATCGAATCAGTGATCATCGAACGCGCATCACGCGAGCATGT

ACAAAGCCGCCTTCGGGCCGGTGTACTTGAGCATGGCACCGTTGACATGCTGCGCGAG

TTGGGCTTGGGTGAGCGCATTGGCAAGATTGGCTTGGAGCAGCATGCTATTGATTTTC

GGTTTGGAGGCGAGTCCCATCGCTTGGACTTTCACCAAGCGACGAATGGACGGTCGGC

ATGGGTGTACCCTCAGCATGAGTTGGTGACTGATTTGATGAATGCACGTATGCAAGCT

GGTGCTGAGATTCTCTTCGAAACTCCCGTAACTCGGATTGACGGACTGCATACCAGCA
 
AACCAACTGTTCACTTCGAGCAAGGAGGCGAGCCGCGTGAGCTCGTCTGTGACTTCGT
GGTAGGTTGTGACGGCTTTCGTGGTGTATGTCGTGATGCTATTCCTGATACCGTACTT
AAAACTTACGATCGTGTGTACCCCTTCGGCTGGCTGGGTATTTTGGCTGAAGCACCGG
CCCCGACTCGCGAAATCACTTGGGGCTGCCACGAAGAAGGATTTGCCATGCTCTCTAT
CCGTTCGCCGAGCGTGACCCGTTTGTATCTCCAGTGCGAACCTGATGATGATCCTGAT
CATTGGAGCGACGACCATATCTGGGCAGAGCTGCACAAGCGGCTTGATGTTCCCGGCA
TGCCCCCCGTTAATGAGGGAAAGATTATGCAAAAAGGTGTGACCGCCATGCGCTCGTT
TTTGGCAGAGCCTATGCAATATGGCAAGCTTTTCCTTGCAGGAGACGCCGCCCACATT
GTGCCCCCAACCGGAGCTAAAGGTCTCAATTCCGCTATGGCGGATGTCAAAGTGCTTG
GTCGTGGCTTGGCTGAAAAGTACCGGCGTGGTGCTGATCACCTTTTGGAACGTTATTC
CCAAATCTGTCTCAAGCGCATGTGGTTGGTACAACGTTTTTCCGCGGGCTTGTGCACG
ATGGTGCACCAATTCCCCGGTGATAACGCTTTTGTGCGGCGCTTGCAACGGGCTGACC
TTGATTACATGACCGGAACGGCGGCGGGACGTCTCGCGTTTTCAGAGAATTTCACTGG
ACTTCCGATTGAATAA
<210> SEQ ID NO:8
   <211> Length 44
   <212> Type DNA
   <213> Organism Polaromonas sp.
<400> Sequence 8
   ATCACCACAGCCAGGATCCGAATTCGCGCACCCAGGTTGGTATC
<210> SEQ ID NO:9
   <211> Length 41
   <212> Type DNA
   <213> Organism Polaromonas sp.
<400> Sequence 9
   TTAAGCATTATGCGGCCGCAAGCTTAGTCGATGGGCAAGCC
<210> SEQ ID NO:10
   <211> Length 27
   <212> Type DNA
   <213> Organism Hylemonella gracilis
<400> Sequence 10
   CTGTGGCGGAAAACTACGTGGGACTGC
<210> SEQ ID NO:11
   <211> 27
   <212> Type DNA
   <213> Organism Hylemonella gracilis
<400> Sequence 11
   GCAGTCCCACGAAGTTTTCCGCCACAG
<210> SEQ ID NO:12
   <211> Length 27
   <212> Type DNA
   <213> Organism Hylemonella gracilis
<400> Sequence 12
   CATCGTTCCTCCGGCTGGCGCAAAGGG
<210> SEQ ID NO:13
   <211> Length 27
   <212> Type DNA
   <213> Organism Hylemonella gracilis
<400> Sequence 13
   CCCTTTGCGCCAGCCGGAGGAACGATG
<210> SEQ ID NO:14
   <211> Length 1185
   <212> Type DNA
   <213> Organism Pseudomonas aeruginosa
<400> Sequence 14 
ATGAAAACACAGGTAGCCATCATTGGTGCGGGTCCAAGTGGGTTGTTGCTCGGCCAGT

TACTCCACAAAGCAGGCATCGATAACGTGATTCTGGAGCGTCAAACGCCGGATTACGT

ACTGGGACGCATTCGCGCGGGAGTGCTGGAACAAGGTATGGTTGATCTGCTGCGTGAA

GCGGGAGTAGATCGCCGGATGGCGCGCGACGGCTTAGTGCATGAAGGTGTCGAGATCG

CTTTCGCTGGGCAACGCCGTCGGATCGACCTGAAACGCCTGTCTGGCGGTAAAACGGT

GACCGTGTATGGCCAGACAGAAGTGACGCGTGACCTGATGGAGGCACGTGAAGCCTGT

GGCGCTACGACCGTTTATCAGGCCGCGGAAGTCCGCTTACACGATTTGCAGGGTGAAC

GTCCGTATGTCACCTTCGAACGGGATGGGGAACGCCTGCGCTTGGACTGCGACTATAT

TGCTGGTTGCGATGGCTTTCATGGCATTTCACGTCAGAGCATTCCGGCAGAACGCTTA

AAAGTGTTCGAACGTGTCTACCCTTTTGGTTGGTTAGGCCTGCTTGCCGATACTCCAC

CCGTTTCGCACGAACTGATCTACGCAAATCATCCGCGTGGCTTTGCCTTGTGCTCCCA

ACGTTCCGCGACGCGCTCTCGTTATTACGTGCAAGTTCCACTCAGTGAGAAAGTTGAG

GATTGGAGCGATGAACGCTTCTGGACCGAACTCAAGGCACGCTTACCCAGTGAGGTAG
 
CCGAAAAGCTGGTGACTGGCCCGTCACTTGAAAAGAGCATTGCACCGTTGCGCTCGTT

TGTTGTCGAACCTATGCAGCATGGCCGCCTGTTTCTGGCGGGAGATGCCGCGCACATT

GTTCCGCCTACTGGCGCGAAAGGGCTGAATCTGGCAGCTTCAGACGTTTCTACCCTCT

ATCGCCTGCTGCTTAAAGCCTATCGTGAGGGTCGTGGGGAACTGCTGGAACGCTATTC

GGCGATCTGTCTGCGCCGTATCTGGAAAGCGGAACGCTTTAGCTGGTGGATGACCAGC

GTGCTGCATCGGTTTCCGGATACCGACGCCTTCAGCCAGCGCATCCAGCAGACCGAAC

TGGAGTACTACCTTGGCTCCGAAGCTGGTCTGGCGACAATTGCGGAAAACTACGTCGG

TCTGCCGTATGAGGAGATTGAGTAA
<210> SEQ ID NO:15
   <211> Length 22
   <212> Type DNA
   <213> Organism Pseudomonas aeruginosa
<400> Sequence 15
   TTCGTCGGTCTGCCGTATGAGG
<210> SEQ ID NO:16
   <211> Length 21
   <212> Type DNA
   <213> Organism Pseudomonas aeruginosa
<400> Sequence 16
   GTTTTCCGCAATTGTCGCCAG
<210> SEQ ID NO:17
   <211> Length 1188
   <212> Type DNA
   <213> Organism Corynebacterium glutamicum
<400> Sequence 17 
ATGAATCACGTACCTGTGGCAATCATTGGAGCTGGCCCCGCGGGTCTGACGCTGGCCC
ACCTGCTGCATCTTCAAGGCGTCGAGAGCATTGTGTTCGAGTCTCGGACGCGCAAGGA
TGTAGAAGAGACTGTTCGTGCTGGAATCTTGGAACAAGGCACGCTCAATTTGATGCGT
GAAACCGGTGTCGGAGCACGTATGGAAGCCGAGGCTGACCATGACGAGGCAATCGACA
TCTCTATCAACAATGAGCGCACGCGCATCCCGCTTACCGAGTTGACGGGCCACAAAGT
AGCTATTTACCCACAGCATGAATATCTGAAGGATTTCATCGCAAAGCGCATCGAGGAC
GGTGGAGAACTCTTGTTTACCACGACTGTAGACAGCGTTGAAAACTACGAAGGAGACC
TGGCTAAGGTTACGTATACCGAAGCAGACGGATCAAGCACTACTATTACCGCTGACTA
CGTTATCGCCGCGGACGGCTCGAACTCGCCTTATCGCAAGCTCATTACCGAAGATGGC
GGTGTACGTGCGCGCCATGAATATCCATACGCTTGGTTCGGTATCCTCGTAGAGGCCC
CCAAAACCCAAAAGGAGCTGATTTATGCGACGCACCCGGAAGGCTTCGCCCTGATCAG
CACGCGTACTGACGAAATCCAGCGGTATTATCTGCAATGCAATCCAGATGATACCCCC
GACATGTGGCCGGACGATCGCATTTGGGAGCAGTTGCACCTTCGTGCCGATAGCCCTG
GAATTACTGTGTCCGAGGGACGGATTTTCGACAAAGCGGTTCTGCGTTTCTGTTCCGC
GGTCACGGAGCCTATGCAGAAAGGCCGCCTGTTTTTGGCGGGAGATGCGGCTCATACC
GTACCGCCTACGGGTGCAAAAGGCCTCAATCTGGCGGTAGCTGATGTATCCGTCCTGG
CACCCGCGTTGGTTCGTGCATTGAAGAAGGATACCGGTCTTCTGGATTCCTACAC
CAGCCTGGCAGTTCCACGCATCTGGAAAGCTCAACACTTTTCTTATTGGATGTCGTCA
ATGCTCCACGCCGTGCCCGGAGAGGATCACTTCGCAACGCAACGCCGTTTCGCCGAAC
TTCGTTCTGTCCTGGAGTCTCAGAGCGGACAACGTTACCTCGCTGAGCAATACGTTGG
CCGGGATCTCCCGCGGTTTGAAGTGTAA
<210> SEQ ID NO:18
   <211> Length 43
   <212> Type DNA
<213> Organism Corynebacterium glutamicum
<400> Sequence 18
   ATCACCACAGCCAGGATCCGAATTCAAATCACGTACCTGTGGC
<210> SEQ ID NO:19
   <211> Length 41
   <212> Type DNA
   <213> Organism Corynebacterium glutamicum
<400> Sequence 19
   TTAAGCATTATGCGGCCGCAAGCTTACACTTCAAACCGCGG
<210> SEQ ID NO:20
   <211> Length 402
   <212> Type PRT
   <213> Organism Hylemonella gracilis
<400> Sequence 20 
MSYPTRTQVAIIGAGPSGLMLGALLHKAGIDTVVLERQSGDYVLGRIRAGVLEQVTMD

LMDEIGVGARMHQEGLVHGGFDMLFQGQRHRIDMNRLTGGQNVMVYGQTEVTRDLMDA

RQQAGLTTIYEAANVAIHDFGSGKPRVTYEKDGKTHELQCDFIAGCDGFHGVCRDTVK

KSASASAIREYEKVYNFGWLGLLSDTPPVHHELIYVNSERGFALCSQRSATRSRYYLQ

VPLTDKVEQWSDQAFWDELKLRLDPQAREHLVTGPSIEKSIAPLRSFVTEPLRFGRLF

LAGDAGHIVPPAGAKGLNLAATDVKYLSAALTEFYQGKSEAGINHYSERCLKRIWRAE
 RFSWWFTSLMHRFPENGEIGQKLQEAELDYIVHSETGARSVAENFVGLPLDFGA
<210> SEQ ID NO:21
   <211> Length 391
   <212> Type PRT
   <213> Organism Hydrogenophaga sp.
<400> Sequence 21 
MRTQVGIIGAGPAGLMLAHMLHLEGIESVIIERSKREHVQNRLRAGVLEQGTVEMMRE

LGLGERIGQLGLEQHRIDFRFGGESHPIDFHEATGGRSAWVYPQHEVVKDLMAARTAA

GAQMLFETPVTAIEGIDGTRPVVRFEKDGVAGELHCDFVVGCDGFRGISRRAIPAREG

QVHDRIYPFGWLGILAEAPPATNEVTWGCHEDGFAMMSIRTPSVTRLYLQCEPDENPD

HWSDDRIWSALHKRLDVPGLPPINEGRITQKGVTAMRSFLFEPMQHGRLFLAGDAAHI

VPPTGAKGLNSAMADVKVLARALEQHYQRGSQDLLARYSKTCLKRMWLVQRFSAGLCT

MVHSFAGDSSFVRRLQRADLDYMTGTTAGRLQFSENFTGLPID
<210> SEQ ID NO:22
   <211> Length 391
   <212> Type PRT
   <213> Organism Polaromonas sp.
<400> Sequence 22 
MRTQVGIVGAGPAGLMLSHMLHLEGIESVIIERASREHVQSRLRAGVLEHGTVDMLRE

LGLGERIGKIGLEQHAIDFRFGGESHRLDFHQATNGRSAWVYPQHELVTDLMNARMQA

GAEILFETPVTRIDGLHTSKPTVHFEQGGEPRELVCDFVVGCDGFRGVCRDAIPDTVL

KTYDRVYPFGWLGILAEAPAPTREITWGCHEEGFAMLSIRSPSVTRLYLQCEPDDDPD

HWSDDHIWAELHKRLDVPGMPPVNEGKIMQKGVTAMRSFLAEPMQYGKLFLAGDAAHI
 
VPPTGAKGLNSAMADVKVLGRGLAEKYRRGADHLLERYSQICLKRMWLVQRFSAGLCT

MVHQFPGDNAFVRRLQRADLDYMTGTAAGRLAFSENFTGLPIE
<210> SEQ ID NO:23
   <211> Length 397
   <212> Type PRT
   <213> Organism Limnohabitans sp.
<400> Sequence 23 
MSTTRTQVAIVGAGPSGLLLGQLLFKAGIDAIIVERQSPDYVLSRIRAGVLEQVTMDL
LDEAGVGQRMHKEGLVHTGFDLLFKGARHRIDMDHLTGGKKVIVYGQTEVTRDLMDTR
QAEGLPTVYEAGNVQVHDFDSQKPRVTYEKNGQLHTIECDFIAGCDGFHGVCRASAPK
NAIKEYEKVYPFGWLGVLSDTPPVHHELIYANSTRGFALCSQRSATRSRYYLQVPTTD
KVEQWSDDAFWAELRNRLDPEAREHLVTGPSIEKSIAPLRSFVTEPMRFGRMFLAGDA
AHIVPPTGAKGLNLAATDVKYLSTAFIEFYADQTEAGIDAYSERCLRRVWRAERFSWW
FTSLMHHFPENGDIGQKFQDAELDYLIHSEAGSRTMAENYVGLPLNFGE
<210> SEQ ID NO:24
   <211> Length 391
   <212> Type PRT
   <213> Organism Rhodoferax sedimins
<400> Sequence 24 
MRTQVGIVGAGPAGLMLSHMLHLEGIESVIIERASREHVQSRLRAGVLEHGTVDMLKE

LGLGERISTLGLEQHAIDFRFGGESHRLDFHQATNGRRTWVYPQHEVVTDLMKARMQA

GAQILFETSVTRIEGLQTRKPTIHFEQGGEPRELQCDFIVGCDGFRGVCRDAIPDSVL

KIYDRVYPFGWLGILAEAPAPTNEITWGCHEDGFAMLSIRSPSVTRLYLQCEPNEDAD
 
RWSDGRIWAELHKRLDVPGMPPVNEGKITQKGVTAMRSFLAEPMQYGRLFLAGDAAHI

VPPTGAKGLNSSMADVKVLGRGLVEQYKRGVADILDRYSQICLKRMWLVQRFSAGLCT

MVHQIPGDNAFVRRLQRADLDYMTGTVAGRLAFSENFTGLPIE
<210> SEQ ID NO:25
   <211> Length 391
   <212> Type PRT
   <213> Organism Xylophilus ampelinus
<400> Sequence 25 
MRTQVGIVGAGPAGLMLAHLLRREGIDAVVIERAAREHVRTRLRAGVLEQGTVEMLRE

AGVGGRIDAVGMEMHAIDFRFGGRSHRLDFHEASGGRRAWVYPQHEVVTDLMSACDAG

DVPILYEAPVERIEGLEDDRARIVFGQDGAAGEITCDFVAGCDGFRGVSRGSMPAGIA

RGYDRIYPFGWLGILADAPPASPDVTWGCSDRGFAMMSMRSPTVTRLYLQCEPDEDPD

AWSDDRIWSELHRRLDVEGMPSLREGPIRDKGVTAMRSFLSEPMQHGRLFLAGDAAHI

VPPTGAKGLNSAMADIKVLAAALVDHYRHGRSDRLATYSERCLRRMWLVQRFSAALCT

MVHQFPGQNEFVRRLQRADLDYMTGTHAGRLQFAENFTGLPIE
<210> SEQ ID NO:26
   <211> Length 390
   <212> Type PRT
   <213> Organism Ramlibacter henchirensis
<400> Sequence 26 
MRTQVGIVGAGPAGLMLAQILHLQGIDSVLIERASQEHVRSRLRAGVLEQGTVEMMRE
FGVGERVMKVGLRQRAIDFRFNGESHPIDFESVCGRNTWVYPQHEVVSDLMAARERSG
LPVVYDAPVSRIEGLDGGRPVIHYERDGEPLRLECDYVAGCDGFRGISRSWIPADRLK
 
VYDRIYPFGWLGILADAKPAIQDITWGCHEDGFAMMSIRSPTVTRLYLQCEPDENPDD

WPDDRIWAELHKRLDVPGLPRLEEGRITQKGVTAMRSFLAEPMQYGRLFLAGDAAHIV

PPTGAKGLNSALADVKVLARALTEQYRNENGQWLERYSDICLKRMWLVQRFSSGLCTM

THQFRGDEPFVRRLQRADLEYMTMHAPGRLEFSENFTGLPVE
<210> SEQ ID NO:27
   <211> Length 374
   <212> Type PRT
   <213> Organism Comamonas phosphati
<400> Sequence 27 
MSHMLHLAGIESVVLERAGREHVRTRLRAGVLEQGVVEMLRELGLGERLDQLALKQSA
LDFRFDGASRRIDFEAEIGRNVWVYPQHEVVTDLMNARQKAGAAIFYEAPVQRIDGLE
GERPVLHFEHQGESCRLECDYVAGCDGFRGVSRSAIPADKLKVFDRIYPFGWLGILAE
APAATNEIVWGCHDGGFAMQSIRSPSVTRLYLQCAPDENPDHWSDDRIWSELHKRLDV
EGMPPVNEGRVTQKGVTAMRSFLAEPMQWGRLFLAGDAAHIVPPTGAKGLNSALADIK
VLARALVAHYREGADAHLARYSDTCLRRMWLVQRFSAGLCTMTHQFPGQNPFVQRLQR
TDLDYMTGTRAGRLQFSENFTGLPVE
<210> SEQ ID NO:28
   <211> Length 394
   <212> Type PRT
   <213> Organism Pseudomonas aeruginosa
<400> Sequence 28 
MKTQVAIIGAGPSGLLLGQLLHKAGIDNVILERQTPDYVLGRIRAGVLEQGMVDLLRE

AGVDRRMARDGLVHEGVEIAFAGQRRRIDLKRLSGGKTVTVYGQTEVTRDLMEAREAC
 
GATTVYQAAEVRLHDLQGERPYVTFERDGERLRLDCDYIAGCDGFHGISRQSIPAERL
KVFERVYPFGWLGLLADTPPVSHELIYANHPRGFALCSQRSATRSRYYVQVPLSEKVE
DWSDERFWTELKARLPSEVAEKLVTGPSLEKSIAPLRSFVVEPMQHGRLFLAGDAAHI
VPPTGAKGLNLAASDVSTLYRLLLKAYREGRGELLERYSAICLRRIWKAERFSWWMTS
VLHRFPDTDAFSQRIQQTELEYYLGSEAGLATIAENYVGLPYEEIE
<210> SEQ ID NO:29
   <211> Length 395
   <212> Type PRT
   <213> Organism Corynebacterium glutamicum
<400> Sequence 29 
MNHVPVAIIGAGPAGLTLAHLLHLQGVESIVFESRTRKDVEETVRAGILEQGTLNLMR

ETGVGARMEAEADHDEAIDISINNERTRIPLTELTGHKVAIYPQHEYLKDFIAKRIED

GGELLFTTTVDSVENYEGDLAKVTYTEADGSSTTITADYVIAADGSNSPYRKLITEDG

GVRARHEYPYAWFGILVEAPKTQKELIYATHPEGFALISTRTDEIQRYYLQCNPDDTP

DMWPDDRIWEQLHLRADSPGITVSEGRIFDKAVLRFRSAVTEPMQKGRLFLAGDAAHT

VPPTGAKGLNLAVADVSVLAPALVRALKKKDTGLLDSYTSLAVPRIWKAQHFSYWMSS

MLHAVPGEDHFATQRRFAELRSVLESQSGQRYLAEQYVGRDLPRFEV
<210> SEQ ID NO:30
   <211> Length 408
   <212> Type PRT
   <213> Organism Corynebacterium ammoniagenes
<400> Sequence 30 MGTPTNSDSSHTPVAIIGAGPAGLMLSHLLHLEGVESVVIEKQSREEVESTVRAGILE 
QGTIDLLRKTGVGERLDREAEIDEGISISIAGESHRIDFAKYTGKQVAVYPQHEVLID
LIAKRLSDDGELWFDTEVVSIADHESDNPKVHYRTADGTEGVLSADFVVGADGSKSLA
RKLITDDGGLRMKHEYPFAWFGIMVNAPQTAPEVIYATHPEGFALISTRSENVQRYYL
QCNPDDTPDMWSDDRIWEQLHLRADSDTVTVSEGEITDKAVLRFRSAVTDPMQRGRLF
IAGDAAHTVPPTGAKGLNLAMADVCALAPSLVRAVKKSDTSLLDTYSERALPRVWKTQ
HFSYWMSSMLHSVPAENEMSTLFQTNRRLAELSTVVTSDAGRQLLAQQFVGWDFPSIE
AL
<210> SEQ ID NO:31
   <211> Length 404
   <212> Type PRT
   <213> Organism Corynebacterium callunae
<400> Sequence 31 
MNHIPVAIIGAGPAGLTLAHLLHLQGIESIVFEKRTRKEVEETVRAGILEQGTLNLMR

ETGVGARMEAEADHDEAIDISINGERIRIPLTEITGHKVAIYPQHEYLKDFIAKRLED

GGELLFSTTVDAVEGTGGTGGTEGFDNDKVKVVYTEADGSSTTISADYVIAADGSSSP

YRKIITEGSGVRARHEYPYAWFGILVEAAKTQKEVIYATHPEGFALISTRTDTVQRYY

LQCDPNDTPDMWSDDRIWEQLHLRADSPGITVSEGKIFDKAVLRFRSAVTEPMQKGRL

FLAGDAAHTVPPTGAKGLNLAVADVSVLAPGLVRALKKKDSALLDDYTKLALPRVWKA

QHFSYWMSSMLHAVPDEDYFATQRRFAELHSVLGSEAGQRYLAEQFVGRDLPRFEV
<210> SEQ ID NO:32
   <211> Length 399
   <212> Type PRT
   <213> Organism Corynebacterium efficiens
<400> Sequence 32 
MNHVPVAIIGAGPAGLTLAHLLHLQGIESVVFESRSRQDVEETVRAGILEQGTLNLMR
ETGVGERMEREADHDEAIDIAINNENIRIPLTELTGHKVAIYPQHEYLKDFIAKRIED
GGELLFETTVDSVENFEGDAQENKATITYTGVDGTTRQLTADYVVAADGSNSPYRKII
TDDGGIRARHEYPYAWFGILVEAPKTQKEVIYATHPEGFALISTRTDTVQRYYLQCDP
NDTPEMWSDDRIWEQLHLRADSPGITVSEGKIFDKAVLRFRSAVTEPMQKGRLFIAGD
AAHTVPPTGAKGLNLAVADVSVLAPGLTRAIKRNDTGLLDDYTRLTVPRIWKAQHFSY
WMSSMLHAVPGEDHFATQRRFAELRSVLESEAGQRYLAEQFVGRDLPRFDY
<210> SEQ ID NO:33
   <211> Length 2241
   <212> Type DNA
   <213> Organism Corynebacterium glutamicum
<400> Sequence 33 
ATGCGTTTTGATCTCCATTCTGTAGTTATTTCCACATTGGTTTTCGCTGCGGTTGCTT

TGGTATGGCGCTTGTTTTTTGTCGGTGGTTGGCTTGTACGTCGGCGTAAAGCACGTAT

CCGCAGGCAGACGCTCGCGGATGAAGAGCGGGCAGAAAATGCTGAGGTATCTGCAGGG

GAGCCTGCCGAATCGAGCACAAACGAAGCAGCCGAATCTGAATCTGAAACCTCGGAGC

GTCGCGGAATCTGGCGCGTGATCTTTGATTACATGCGCGACGGTGGCATCTTGGATCA

CCGTTGGCTGCTGCCTGCCGCAGGTGCTATCACTGGTGCGTGGCTGATCATTGATCGT

GCCGTTGATCTGCTCTTGAGCACCGAGCATGGTTTGGGCGATATCGTCCAAGGCTGGG

ATGTCCATTGGCATGCTTCGACTGTCCGTTTTATAGATGAGACCGGCATTGCGTCATC

CACGATGATGGGGCAGCTGCGCAATATTGAAACGCAGCAAGATCTGTTCTACCCAAGC

GCATGGCATGCTGGTGCATGGGTGCTGTCGGATGTCGGAAATCTGACGATTGTTGAAG

CCACCAACCTCACTGGCATTGTGCTGTCCGGATTGTTGCTGCCGTTAGCTGTTGCACT
 
GATTGCATGGCGGATGATCAACAATCGTGGACTGACCGCGCAGATTGGTGCGGGCTTT
GCTGGACTGATCACCATTGCCTCTCCGGTACTGTTCTGGGTTGGTAACTACGTGGGTG
CGTGGCCTTATGTTGCTGCGATCGGTGCTTCAGGTGTGGTGCTTGCGCTGTTTATGTC
CACTCCGTCTGTGCCGGTAAGAATCTTTGCCGCAGCATTGGCGTTCATGGGTATGTTC
CAGCTGCATCCAGCCCCATCCACCATCGTGATCATGGTGTTGCTGCTGTGGTGGCTGC
TCAAACTCGTGGTGGTTCCAAGCCAGAAAGTGAAGGGCTGGAAGGCGGGCATCGGTAT
CCGTTTGAAGGATGTCGGCATCCTGGCCATCACGGGCATCATCGGTGTGCTCTTCATG
CTGCCTCAGGTGATTTCAGGTTCCGAACAAACCGAAGATGTGCTGTCATATTCTGCTG
AGGAACAAGTCACCCGCAGCGAGTCCTGGTTGGTGTCTATTTTCATGGAGACCCGCCA
TGTTGATTTCTTCGGAAATATTGACATCGTCCCAGTGCTGGTATTCGCAGCAATCGGT
GGCGTGGTTGCTTTGGTGTGGCGCGGAAACTTGTGGGCGCCGGTGTTTTACTTCGCCA
GCGTTGCGTTGACCGCTAACTCGCTGAAGCCTTTTGAAGAGCCGTGGGGTGATTGGCT
CAACATCGTGGGCGGTCTGCATTACTCCACAGGACACCGTTTGATCATGCCTGTCGCC
ATGTTCACTTTTGCTGCCGCAGGTATCGGCGCTGCCGCAGTGATCCGTTTGATCTGCT
TGGGACCAATAAAGAAGTTCACCACTGTTTCCGGTGTTGTTTCTGTGGTGATGGCTCT
TGTTGTGGCTGTGCCATTGCAGACTTGGGCGAAGGATTTTGTAGAGGAAGGATCCGAA
ACCACAATCCTTGCGCCACACAATGATGAACGTATGGTGAGCAACAACGACTTGGCTG
CCTGGGACTGGTTAATCCAACAGCCAGGTGGAGCTGACATGAACATCATGGGTGACCC
CGCAGATGGTAACGGCTGGATGTATGCCTACAACGGCTTGCACTCCGTGGCCCGCCAC
TATGCATGGCCAGCAGCAGGCGAAGGCTCTGCCACCGCGATGCTGTTCTGGTGGCCTC
AACTTCTAGGTGTGGGCACCGATGAAAACCCAGATCAAGTCAACGATGTGGATCAGGC
TGCTCGTGATCTCAACGTCGGCTACTTCATGATCAGTCCGTGGACGTTCTGGGATTTC
CAGATCCCCAACTTCCGCCAGATCGATCTGCTGTGGCAAACCCCAGGCGTGACACCGG
TGTACAAGAAGGGCGACTCGGTGATCTTCGCAGTCAACGATATGTTCACTGACGCCGA
ACTGGATCAGATGCGTGCACCTGGTAATTCTCCAGAACCACTGCCAGAGCTTCCTACC
 
TTGGGCGAGCTTGGGTTGGCTGAAACTGAAGACGAGGTAGATCAGACTTATTACCATC

GTCCAACGGTTCCTGCTGGTGTGAACTCAGAGATGCCTTCAGCCGAAACTCTGTATGC

ACCGGATCCAACGAAGCCGCATACGGTCCCTAACTAA
<210> SEQ ID NO:34
   <211> Length 26
   <212> Type DNA
   <213> Organism Corynebacterium glutamicum
<400> Sequence 34
   GAGCTCATGCGTTTTGATCTCCATTC
<210> SEQ ID NO:35
   <211> Length 33
   <212> Type DNA
   <213> Organism Corynebacterium glutamicum
<400> Sequence 35
   GAGCTCCTCTAGATTAGTTAGGGACCGTATGCG
<210> SEQ ID NO:36
   <211> Length 125
   <212> Type DNA
   <213> Organism Escherichia coli
<400> Sequence 36 
TTCGATTTATTCAACAAAGCCACGTTGTGTCTCAAAATCTCTGATGTTACATTGCACA

AGATAAAAATATATCATCATGAACAATAAAACTGTCTGCTTACATAAACAGTAATACA
 AGGGGTGTT
<210> SEQ ID NO:37 (sequence of HgPobA_TY)
   <211> Length 1209
   <212> Type DNA
   <213> Organism Hylemonella gracilis
<400> Sequence 37 
ATGTCTTACCCTACGCGGACCCAGGTAGCTATCATCGGCGCAGGTCCGTCAGGCCTGA

TGTTGGGCGCTCTTCTCCACAAGGCTGGTATTGATACGGTTGTTCTCGAACGTCAGTC

CGGCGATTATGTTTTGGGACGTATCCGCGCCGGAGTACTCGAACAAGTGACTATGGAT

CTGATGGATGAGATTGGAGTCGGCGCCCGTATGCACCAGGAGGGCTTGGTTCACGGTG

GTTTTGACATGCTGTTTCAAGGCCAACGGCATCGTATTGATATGAATCGGTTGACTGG

CGGACAAAATGTAATGGTCTACGGTCAGACCGAAGTCACGCGGGATCTTATGGATGCT

CGCCAACAAGCCGGTTTGACCACGATCTACGAGGCTGCAAACGTGGCGATTCATGATT

TCGGAAGCGGCAAACCTCGGGTCACGTACGAGAAGGACGGTAAGACCCACGAGTTGCA

GTGTGACTTTATTGCAGGTTGTGACGGCTTTCACGGCGTATGCCGGGACACGGTTAAA

AAGAGCGCTTCTGCGTCTGCGATCCGGGAGTATGAAAAAGTGTACAACTTTGGCTGGC

TGGGTCTCTTGTCTGATACGCCGCCGGTTCATCATGAGcTgATCTATGTAAATTCTGA

GCGTGGTTTCGCACTGTGCAGCCAGCGCTCTGCGACCCGTTCTCGTTACTATCTTCAG

GTGCCCCTTACCGATAAAGTAGAACAATGGAGCGACCAAGCTTTCTGGGACGAACTGA

AACTGCGGCTTGATCCTCAAGCCCGTGAACATTTGGTTACCGGTCCATCAATTGAAAA

GTCTATCGCACCGCTCCGGTCATTCGTTACCGAACCCCTGCGGTTTGGCCGTCTCTTC

CTGGCCGGCGATGCGGGACACATTGTCCCACCTgCcGGAGCCAAGGGACTTAATCTGG

CAGCCACTGATGTGAAGTACTTGAGCGCGGCCCTCACGGAATTTTATCAGGGAAAATC

CGAGGCTGGCATCAACCACTACTCTGAACGCTGTCTGAAGCGTATCTGGCGTGCAGAA
 
CGGTTTTCCTGGTGGTTTACTTCACTCATGCATCGTTTCCCGGAGAACGGAGAGATCG

GCCAGAAGCTTCAGGAAGCAGAGCTCGATTACATTGTCCACTCGGAAACCGGAGCGCG

TTCGGTTGCCGAAAACTTTGTTGGACTCCCTCTGGATTTCGGTGCAtaa
<210> SEQ ID NO:38
   <211> Length 27
   <212> Type DNA
   <213> Organism Escherichia coli
<400> Sequence 38
   GAGCTCATGCGCACCCAGGTTGGTATC
<210> SEQ ID NO:39
   <211> Length 26
   <212> Type DNA
   <213> Organism Hydrogenophaga sp.
<400> Sequence 39
   GTCGACTTAGTCGATGGGCAAGCCCG
<210> SEQ ID NO:40
   <211> Length 26
   <212> Type DNA
   <213> Organism Polaromonas sp.
<400> Sequence 40
   GTCGACTTATTCAATCGGAAGTCCAG
<210> SEQ ID NO:41
   <211> Length 26
   <212> Type DNA
   <213> Organism Hylemonella gracilis
<400> Sequence 41
   GTCGACTTAGGCACCGAAGTCGAGGG
<210> SEQ ID NO:42
   <211> Length 25
   <212> Type DNA
   <213> Organism Corynebacterium glutamicum
<400> Sequence 42
   GTCGACTTACACTTCAAACCGCGGG

## Description

### Technical Field

The present technology relates to a microbial strain obtained by introducing a gene related to a specific aromatic hydroxylase, a protein which is a specific aromatic hydroxylase and a method for producing gallic acid using the microorganism or the protein.

### Background Art

Gallic acid (GA) is a phenolic compound contained in many plants, such as galls produced by *Schlechtendalia chinensis,* galls produced by gall wasps, Hamamelis, tea leaves and oak bark. Gallic acid has three hydroxy groups in the molecule and thus exhibits a high antioxidative action and is used as an antioxidant. Moreover, derivatives of gallic acid are used as adhesives, coating agents and products related to electronic parts. In addition, esters of gallic acid are also widely used as additives for many foods. The demand for gallic acid is estimated to be 8000 tons or more per year, and the demand is increasing.

Gallic acid is currently produced industrially from plant raw materials including galls produced by *Schlechtendalia chinensis* and galls produced by gall wasps. Thus, the supply amount of the raw materials per harvest is not stable, which is a cause for the increase in the price. To meet the increasing demand for gallic acid and stably produce gallic acid at a low price, a production method through microbial fermentation using sugar or the like as the substrate is expected.

The production method through microbial fermentation has low environmental burden compared to that of a production method using chemical synthesis because sustainable plant biomass such as starch and cellulose can be used without the use of organic solvents, heavy metals, strong acids and strong alkalis. Moreover, because stable production is enabled by regulating the culture condition, the subject is expected to be provided at a lower price than that of production using plant cells.

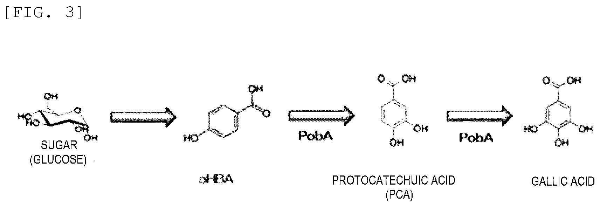

In the production pathway of gallic acid from sugar (glucose) using a microorganism shown in FIG. 3, however, p-hydroxybenzoate hydroxylase (PobA), which produces protocatechuic acid (PCA) from p-hydroxybenzoic acid (pHBA), is known, but no enzyme which can produce gallic acid selectively from protocatechuic acid, which is a precursor of gallic acid, is known.

For example, PTL 1 below discloses a method for producing gallic acid from protocatechuic acid using a microorganism that is derived from *Pseudomonas putida* KT2440 or *Novosphingobium aromaticivorans* DSM 12444 and that expresses p-hydroxybenzoate hydroxylase (PobA), which is an enzyme that produces protocatechuic acid from p-hydroxybenzoic acid, but the ratio of gallic acid to protocatechuic acid is not increased sufficiently because the reaction for producing protocatechuic acid from p-hydroxybenzoic acid advances dominantly over the reaction for producing gallic acid from protocatechuic acid.

### Citation List

### Patent Literature

PTL 1: JP2009-065839A

### Summary of Invention

### Technical Problem

Thus, a main object of the present technology is to provide a protein having protocatechuic acid 5-oxidization activity which can produce gallic acid from protocatechuic acid efficiently and a microbial strain which expresses the protein.

### Solution to Problem

As a result of intensive study, the present inventors have found that it is useful to use a protocatechuic acid 5-hydroxylase derived from a microorganism of the family *Comamonadaceae* or a protein having an identity of 70% or more with the amino acid sequence of the protocatechuic acid 5-hydroxylase for efficient production of gallic acid from protocatechuic acid.

That is, the present technology provides the follows.
[1] A microbial strain obtained by introducing a gene encoding a protocatechuic acid 5-hydroxylase derived from a microorganism of the family *Comamonadaceae* or a protein having an identity of 70% or more with the amino acid sequence of the protocatechuic acid 5-hydroxylase.
[2] The microbial strain according to [1], wherein the microorganism of the family *Comamonadaceae* is a microorganism selected from the genus *Hylemoenella,* the genus *Polaromonas* and the genus *Hydrogenophaga.*
[3] The microbial strain according to [1] or [2], wherein the microbial strain is a microbial strain selected from the genus *Escherichia,* the genus *Rhodococcus,* the genus *Acinetobacter,* the genus *Bradyrhizobium,* the genus *Corynebacterium,* the genus *Pseudomonas,* the genus *Rhodopseudomonas,* the genus *Sinorhizobium,* the genus *Brevibacterium,* the genus *Novosphingobium* or the genus *Ralstonia.*
[4] A method for producing gallic acid from protocatechuic acid through fermentation using the microbial strain according to any of [1] to [3].
[5] A method for producing gallic acid through fermentation using the microbial strain according to any of [1] to [3] in a medium containing glucose.
[6] The method for producing gallic acid according to [4] or [5], wherein the fermentation is conducted using a medium having pH 6.0 or less.
[7] The method for producing gallic acid according to any of [4] to [6], wherein the fermentation is conducted under a condition of 15°C to 45°C.
[8] A method for producing gallic acid from protocatechuic acid using a protocatechuic acid 5-hydroxylase derived from a microorganism of the family *Comamonadaceae* or a protein having an identity of 70% or more with the amino acid sequence of the protocatechuic acid 5-hydroxylase.
[9] A protein having an amino acid sequence in which a leucine at positions 199 to 209 has been replaced with a valine or a glycine in a protocatechuic acid 5-hydroxylase derived from a microorganism of the family *Comamonadaceae* or a protein having an identity of 70% or more with the amino acid sequence of the protocatechuic acid 5-hydroxylase.
[10] A protein having an amino acid sequence in which a threonine at positions 294 to 304 has been replaced with an alanine in a protocatechuic acid 5-hydroxylase derived from a microorganism of the family *Comamonadaceae* or a protein having an identity of 70% or more with the amino acid sequence of the protocatechuic acid 5-hydroxylase.
[11] The protein according to [9] or [10], wherein a tyrosine at positions 385 to 395 has been further replaced with a phenylalanine, a valine or an alanine.

### Advantageous Effects of Invention

The microbial strain according to the present technology can improve the yield of gallic acid from protocatechuic acid and can produce gallic acid efficiently.

Through fermentation using the microbial strain according to the present technology, the yield of gallic acid from protocatechuic acid improves, and gallic acid can be produced efficiently.

Using the protein having protocatechuic acid 5-hydroxylation activity according to the present technology, the yield of gallic acid from protocatechuic acid improves, and gallic acid can be produced efficiently.

The effects of the present technology are not limited to the effects described here and may be any effects described in the present description.

### Brief Description of Drawings

[FIG. 1] A figure showing the results of a molecular phylogenetic analysis of PobA protein.
[FIG. 2] A figure showing the activities of producing gallic acid of the protocatechuic acid 5-hydroxylases according to the present technology.
[FIG. 3] A figure showing the production pathway of gallic acid from sugar using a microorganism.

### Description of Embodiments

Preferable embodiments of the present technology are explained below. However, the present technology is not limited only to the preferable embodiments below and can be freely changed in the scope of the present technology.

The microbial strain of the present technology is a microbial strain obtained by introducing a gene encoding a protocatechuic acid 5-hydroxylase derived from a microorganism of the family *Comamonadaceae* or a protein having an identity of 70% or more with the amino acid sequence of the protocatechuic acid 5-hydroxylase.

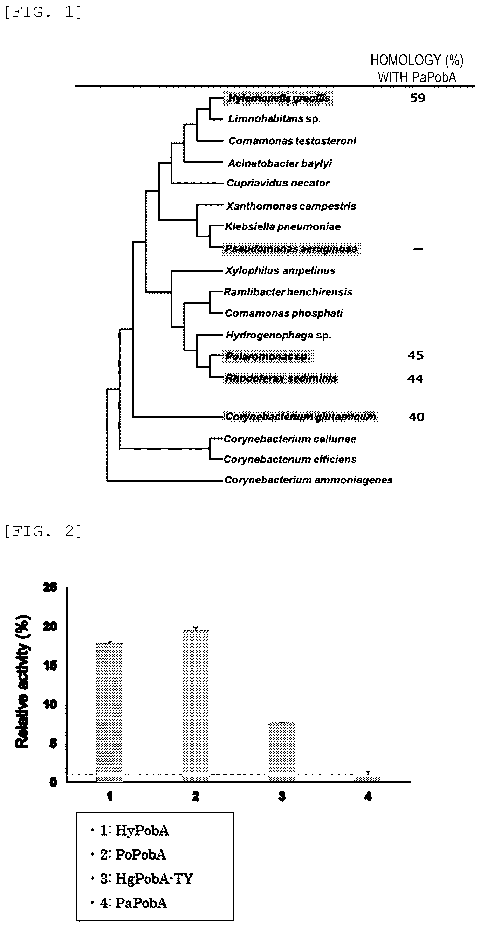

The microorganism of the family *Comamonadaceae* which is the origin of the protocatechuic acid 5-hydroxylase of the present technology is explained based on FIG. 2. Using the protocatechuic acid 5-hydroxylase derived from the microorganism of the family *Comamonadaceae,* protocatechuic acid can be converted to gallic acid at high efficiency.

The microorganism of the family *Comamonadaceae* is not particularly limited as long as the microorganism has the above feature, but of the genera cited in the results of the phylogenetic analysis in FIG. 1, the genus *Hylemonella,* the genus *Limnohabitans,* the genus *Rhodoferax,* the genus *Xylophilus,* the genus *Ramlibacter,* the genus *Polaromonas* and the genus *Hydrogenophaga* are preferable, and the genus *Hylemonella,* the genus *Polaromonas* and the genus *Hydrogenophaga* are particularly preferable.

Amino acid sequence information of *Hylemonella* gracilis-derived PobA (HgPobA) (SEQ ID NO: 20)
Amino acid sequence information of *Limnohabitans* sp.-derived PobA (SEQ ID NO: 23)
Amino acid sequence information of *Rhodoferax* sedimins-derived PobA (SEQ ID NO: 24)
Amino acid sequence information of *Xylophilus ampelinus*-derived PobA (SEQ ID NO: 25)
Amino acid sequence information of *Ramlibacter henchirensis*-derived PobA (SEQ ID NO: 26)
Amino acid sequence information of *Comamonas phosphati*-derived PobA (SEQ ID NO: 27)
Amino acid sequence information of *Hydrogenophaga* sp.-derived PobA (HyPobA) (SEQ ID NO: 21)
Amino acid sequence information of *Polaromonas* sp.-derived PobA (PoPobA) (SEQ ID NO: 22)

A gene encoding a protein having the same amino acid sequence as that of the protocatechuic acid 5-hydroxylase derived from a microorganism of the family *Comamonadaceae* to be expressed in the microbial strain of the present technology may be introduced, but a gene encoding a protein in which the amino acid sequence has been changed in the scope in which the protocatechuic acid 5-hydroxylase can maintain the activity of hydroxylating protocatechuic acid and producing gallic acid may also be introduced. A protein having an identity of preferably 70% or more or having an identity of more preferably 80% or more, further preferably 90% or more, further preferably 95% or more, further preferably 97% or more, further preferably 98% or more, further preferably 99% or more, further preferably 99.5% or more, further preferably 99.7% or more, with the amino acid sequence of the protocatechuic acid 5-hydroxylase can maintain the activity of hydroxylating protocatechuic acid and producing gallic acid and thus is believed to have a high homology with the protocatechuic acid 5-hydroxylase according to the present technology.

In the present technology, the form of the gene to be introduced to the microbial strain is not particularly limited, but for example, a polynucleotide encoding the protocatechuic acid 5-hydroxylase or the protein described above, a vector containing the polynucleotide in the sequence or the like can be used.

The polynucleotide encoding a protein such as the protocatechuic acid 5-hydroxylase in the present technology preferably includes a regulatory region which is linked to the upstream region (the region at the 5' end of the polynucleotide chain) of the region encoding the protein such as the protocatechuic acid 5-hydroxylase in such a manner that the regulatory region can operate. The regulatory region preferably includes a promoter and may further include a cis-element which improves the transcriptional activity of the promoter, a terminator or the like. In addition, when the polynucleotide includes a selection marker gene, such as a drug-resistant gene and an auxotrophic marker gene, the microbial strain to which the gene has been introduced can be found by screening efficiently.

Furthermore, by providing a restriction enzyme recognition sequence at both ends of the polynucleotide encoding the protein such as the protocatechuic acid 5-hydroxylase in the present technology, the polynucleotide can also be introduced to a vector by the restriction enzyme method.

The kind of the vector which can be used in the present technology is not particularly limited and may be any vector such as a plasmid, a cosmid, a phasmid, a phage, a transposon and a BAC vector. Moreover, the vector may be a vector for introducing to the chromosome in the cell of the microbial strain to which the gene is introduced or a vector which is maintained outside the chromosome, but the vector is preferably capable of amplifying in the cell of the microbial strain. The vector which can be used in the present technology is not limited but is preferably a vector for a bacterium, more preferably a vector for the microbial strain to which the gene is introduced.

In the present technology, the microbial strain to which the gene is introduced is not particularly limited, but a microbial strain belonging to the genus *Escherichia,* the genus *Rhodococcus,* the genus *Acinetobacter,* the genus *Bradyrhizobium,* the genus *Corynebacterium,* the genus *Pseudomonas,* the genus *Rhodopseudomonas,* the genus *Sinorhizobium,* the genus *Brevibacterium,* the genus *Novosphingobium* or the genus *Ralstonia* is preferable, and the genus *Escherichia* and the genus *Corynebacterium* are particularly preferable.

The method for introducing the gene to the microbial strain is not particularly limited, but a general transformation method, for example, a known transformation technique such as the method using calcium ion [Proc. Natl. Acad. Sci. USA, 69, 2110 (1972)], the electroporation method [Nucleic Acids Res., 16, 6127 (1988)], the transformation method, the transfection method, the conjugation method, the protoplast method, the particle gun method and the Agrobacterium method, can be applied.

Gallic acid can be suitably produced from protocatechuic acid through fermentation using the microbial strain of the present technology. The fermentation using the microbial strain of the present technology is not particularly limited but can be advanced suitably, for example, by adding a culture liquid obtained by culturing the microbial strain of the present technology as a preculture to a medium used for fermentation.

As the medium used for preculturing the microbial strain of the present technology, a medium which is generally used for the microbial strain to which the gene is introduced can be suitably used. For example, when the microbial strain to which the gene is introduced is of the genus *Escherichia,* the microbial strain can be suitably cultured using LB medium, M9 medium or the like, and the condition can be a culture temperature of 15°C to 45°C, a culture period of a day to seven days and the like. When the microbial strain to which the gene is introduced is of the genus *Corynebacterium,* the microbial strain can be suitably cultured using LB medium, CGXII medium (Journal of Bacteriology, 1993, 175:5595-5603) or the like, and the condition can be a culture temperature of 15°C to 45°C, a culture period of a day to seven days and the like.

By conducting the preculture before the step related to the fermentation, gallic acid can be suitably produced from protocatechuic acid efficiently using the microbial strain in the logarithmic growth phase.

The medium used for the fermentation of the microbial strain of the present technology is not particularly limited as long as the medium contains a compound which is a precursor of gallic acid in the production pathway of gallic acid shown in FIG. 3 as a substrate. Here, as the compound contained in the medium which is a precursor of gallic acid, the compound shown in the production pathway of gallic acid shown in FIG. 3 may be directly contained, and for example, a compound such as terephthalic acid, phthalic acid and isophthalic acid, which can be a precursor of protocatechuic acid or the like as the compound shown in the production pathway of gallic acid shown in FIG. 3, may also be contained.

Regarding the substrate contained in the medium used for the fermentation of the microbial strain of the present technology, a medium containing glucose is preferably used for decreasing the production cost of gallic acid. The medium containing glucose can be prepared by adding 1 to 10%, preferably around 3 to 7% glucose to a medium which is generally used for the microbial strain to which the gene is introduced described above.

For example, when the microbial strain to which the gene is introduced is of the genus *Corynebacterium,* the fermentation can be advanced suitably in a medium obtained by adding glucose to CGXII medium or LB medium, under a condition of a culture temperature of 15°C to 45°C, a culture period of a day to seven days and the like. Moreover, when the microbial strain to which the gene is introduced is of the genus *Escherichia,* the fermentation can be advanced suitably in LB medium containing glucose or a fermentation production medium (Scientific reports, 2016, 6.1: 1-9.), under a condition of a culture temperature of 28°C to 37°C, a culture period of a day to seven days and the like.

By adjusting the pH of the medium used for the fermentation of the microbial strain of the present technology low, the used amount of the acid such as sulfuric acid and hydrochloric acid used for crystallizing gallic acid can be reduced in the step of collecting gallic acid from the fermented liquid. Moreover, by adjusting the pH of the medium used for the fermentation of the microbial strain of the present technology low, the risk of inhibition of the progress of the aimed fermentation due to contamination with a strain other than the microbial strain of the present technology can be reduced.

For example, the pH of a typical fermented liquid in culture is 6.5 to 7.5, and the amount of sulfuric acid used here is estimated to be 13 to 16 g/L. The amount of sulfuric acid used can be estimated to be 5 to 8 g/L when the culture liquid has pH 5.0, and the used amount of sulfuric acid can be reduced.

The pH of the medium used for the fermentation of the microbial strain of the present technology is, for example, preferably 6.0 or less, further preferably 5.0 or less.

Moreover, because the protocatechuic acid 5-hydroxylase derived from the microorganism of the family *Comamonadaceae* used in the present technology can be expected to maintain the enzymatic activity also in a temperature range of 37°C or higher, the temperature condition for the fermentation using the microbial strain of the present technology can also be in a high-temperature range of preferably 35°C to 45°C, further preferably 37°C to 40°C.

The protocatechuic acid 5-hydroxylase derived from the microorganism of the family *Comamonadaceae* used in the present technology can be expressed in a system using the microbial strain to which a gene encoding the protocatechuic acid 5-hydroxylase is introduced as described above but may be expressed in an *in vitro* system by adding a reagent such as amino acids to a gene encoding the protocatechuic acid 5-hydroxylase or a transcription product thereof.

In the present technology, gallic acid can also be produced from protocatechuic acid using a protocatechuic acid 5-hydroxylase derived from a microorganism of the family *Comamonadaceae* which has been expressed in a system using the microbial strain above or an *in vitro* system, or using a protein having an identity of 70% or more with the amino acid sequence of the protocatechuic acid 5-hydroxylase.

In this case, the protocatechuic acid 5-hydroxylase or the protein having an identity of 70% or more with the amino acid sequence of the protocatechuic acid 5-hydroxylase can be suitably collected from a culture liquid of the microbial strain, a cell homogenate, a reaction liquid of an *in vitro* system or the like using one of or an appropriate combination of general methods used for protein purification, such as homogenization of cells, centrifugation, ammonium sulfate precipitation, gel chromatography, ion exchange chromatography and affinity chromatography.

As the protocatechuic acid 5-hydroxylase of the present technology, an enzyme having the same amino acid sequence as that of a protocatechuic acid 5-hydroxylase derived from a microorganism of the family *Comamonadaceae* can be used, but an enzyme having a mutation in the amino acid sequence can also be used.

For example, by replacing a leucine located at positions 199 to 209, preferably at position 199 or position 200, from the N-terminus of the polypeptide chain related to the enzyme with a valine or a glycine, the activity in the production of gallic acid from protocatechuic acid sometimes improves.

Moreover, by replacing a threonine at positions 294 to 304, preferably at position 294 or position 302, from the N-terminus of the polypeptide chain related to the enzyme with an alanine, the activity in the production of gallic acid from protocatechuic acid sometimes improves.

Furthermore, by replacing a tyrosine at positions 385 to 395, preferably at position 385 or position 393, from the N-terminus of the polypeptide chain related to the enzyme with a phenylalanine, a valine or an alanine, the activity in the production of gallic acid from protocatechuic acid sometimes improves.

In the present technology, regarding the mutation site in the amino acid sequence of the protocatechuic acid 5-hydroxylase, one of or an appropriate combination of those cited as examples in the above description can be introduced. However, the mutation site is not limited to the above examples, and an enzyme having a mutated amino acid sequence can be designed by replacing any amino acid residue in the polypeptide chain with another amino acid, deleting any amino acid residue in the polypeptide chain or inserting a new amino acid residue at any position in the polypeptide chain.

The enzyme having a mutated amino acid sequence can be obtained, for example, by mutating a gene encoding the protocatechuic acid 5-hydroxylase of the present technology using a known site-specific mutagenesis method. The known site-specific mutagenesis method can be conducted, for example, by a method such as the inverse PCR method and the annealing method using primers for mutation including a nucleotide mutation to be introduced.

The primers for mutation may be designed in such a manner that the primers anneal to a region including the nucleotide sequence encoding the amino acid residue to be mutated in the target gene and include a nucleotide sequence having a nucleotide sequence (codon) encoding the amino acid residue after the mutation instead of the nucleotide sequence (codon) encoding the amino acid residue to be mutated.

In the present technology, when gallic acid is produced from protocatechuic acid through fermentation using the microbial strain, gallic acid is collected from the fermented liquid, and when gallic acid is produced from protocatechuic acid using the protocatechuic acid 5-hydroxylase, gallic acid is collected from the reaction liquid.

The method for collecting gallic acid is not particularly limited, but for example, the fermented liquid or the reaction liquid described above is adjusted to pH 4.0 or less with an acid such as sulfuric acid and hydrochloric acid. The insoluble protein generated by the pH adjustment is removed by a method such as centrifugation and filtration. Crystals of gallic acid which are obtained by cooling the liquid after the removal to 4°C or lower while appropriately stirring the liquid can be suitably collected by a centrifugation or filtration method. Moreover, instead of adjusting to pH 4.0 or less as described above, by mixing the fermented liquid or the reaction liquid described above with an organic solvent such as benzene, toluene, dichloromethane, chloroform, diethyl ether and ethyl acetate and then collecting and evaporating the organic solvent layer, crystals of gallic acid can also be suitably collected.

### Examples

The present technology is explained more specifically below using Examples. Here, the present technology is not limited at all to the contents of the Examples shown below.

### Example 1: Preparation of Protocatechuic Acid 5-Hydroxylases and Measurement of Activities

### (1) Construction of Protocatechuic Acid 5-Hydroxylase-Expressing Plasmids

### <1> Construction of pRSF-HgPobA Plasmid

A polynucleotide encoding *Hylemonella gracilis-*derived PobA was artificially synthesized based on the gene sequence information of *Hylemonella gracilis*-derived PobA (HgPobA) (SEQ ID NO: 1). A polynucleotide fragment was obtained using the polynucleotide as a template by PCR using primers 1 and 2 having the base sequences shown below and KOD One(R) PCR Master Mix (TOYOBO Co., Ltd.).

Primer 1 (SEQ ID NO: 2)
   (5'-atcaccacagccaggatccgaattcAAGCTATCCCACAAGAACTCAGG-3')
Primer 2 (SEQ ID NO: 3)
   (5'-ttaagcattatgcggccgcaagcttAGGCACCGAAGTCGAGGG-3')

The polynucleotide fragment and pRSFDuet-1 were each digested using restriction enzymes EcoRI and HindIII and then reacted and ligated using Gibson Assembly Master Mix (New England Biolabs), and thus a pRSF-HgPobA plasmid was obtained.

### <2> Construction of pRSF-HyPobA Plasmid

By the same procedures, a target pRSF-PoPobA plasmid was obtained based on the gene sequence information of *Hydrogenophaga* sp.-derived PobA (HyPobA) (SEQ ID NO: 4). The base sequences of primers 3 and 4 used are shown below.

Primer 3 (SEQ ID NO: 5)
   (5'-atcaccacagccaggatccgaattcGCGGACCCAGGTCGGCAT-3')
Primer 4 (SEQ ID NO: 6)
   (5'-ttaagcattatgcggccgcaagcTTATTCAATCGGAAGTCCAGTGAAATTCTCTGAAA ACG-3')

### <3> Construction of pRSF-PoPobA Plasmid

By the same procedures, a target pRSF-PoPobA plasmid was obtained based on the gene sequence information of *Polaromonas* sp.-derived PobA (PoPobA) (SEQ ID NO: 7). The base sequences of primers 5 and 6 used are shown below.

Primer 5 (SEQ ID NO: 8)
   (5'-atcaccacagccaggatccgaattcGCGCACCCAGGTTGGTATC-3')
Primer 6 (SEQ ID NO: 9)
   (5'-ttaagcattatgcggccgcaagcTTAGTCGATGGGCAAGCC-3')

### <4> Construction of pRSF-PaPobA Plasmid (Comparison Subject 1)

A polynucleotide encoding *Pseudomonas aeruginosa-*derived PobA was artificially synthesized based on the gene sequence information of *Pseudomonas aeruginosa*-derived PobA (PaPobA) (SEQ ID NO: 14). A polynucleotide fragment was obtained using the polynucleotide as a template by PCR using a primer set having the base sequences shown below and KOD One(R) PCR Master Mix (TOYOBO Co., Ltd.).

### Primer Set for Constructing pRSF-PaPobA Plasmid

5'-TTCGTCGGTCTGCCGTATGAGG-3' (SEQ ID NO: 15)
5'-GTTTTCCGCAATTGTCGCCAG-3' (SEQ ID NO: 16)

The polynucleotide fragment and pRSFDuet-1 were each digested using restriction enzymes EcoRI and HindIII and then reacted and ligated using Gibson Assembly Master Mix (New England Biolabs), and thus a pRSF-PaPobA plasmid was obtained.

### <5> Construction of pRSF-CgPobA Plasmid (Comparison Subject 2)

A polynucleotide encoding *Corynebacterium glutamicum-*derived PobA was artificially synthesized based on the gene sequence information of *Corynebacterium glutamicum*-derived PobA (CgPobA) (SEQ ID NO: 17). A polynucleotide fragment was obtained using the polynucleotide as a template by PCR using a primer set having the base sequences shown below and KOD One(R) PCR Master Mix (TOYOBO Co., Ltd.).

### Primer Set for Constructing pRSF-CgPobA Plasmid

5'-atcaccacagccaggatccgaattcAAATCACGTACCTGTGGC-3' (SEQ ID NO: 18)
5'-ttaagcattatgcggccgcaagcTTACACTTCAAACCGCGG-3' (SEQ ID NO: 19)

The polynucleotide fragment and pRSFDuet-1 were each digested using restriction enzymes EcoRI and HindIII and then reacted and ligated using Gibson Assembly Master Mix (New England Biolabs), and thus a pRSF-CgPobA plasmid was obtained.

### (2) Construction of Plasmid for Expressing Mutant Protein of Protocatechuic Acid 5-Hydroxylase

### <1> Construction of pRSF-HgPobA-T302A/Y393F Plasmid

A polynucleotide fragment was obtained using the pRSF-HgPobA plasmid above as a template by PCR using an Y393F-introducing primer set having the base sequences shown below and KOD One(R) PCR Master Mix (TOYOBO Co., Ltd.).

### Y393F-Introducing Primer Set

5'-CTGTGGCGGAAAACTACGTGGGACTGC-3' (SEQ ID NO: 10)
5'-GCAGTCCCACGAAGTTTTCCGCCACAG-3' (SEQ ID NO: 11)

The obtained polynucleotide fragment was treated with a restriction enzyme DpnI and then connected into a cyclic form using T4 Polynucleotide kinase (Takara Bio Inc.) and Ligation high Ver. 2 (TOYOBO Co., Ltd.), and the obtained cyclic polynucleotide was named a pRSF-HgPobA-Y393F plasmid.

Then, a polynucleotide fragment was obtained using the obtained plasmid, pRSF-HgPobA-Y393F, as a template by PCR using a T302A-introducing primer set having the base sequences shown below and KOD One(R) PCR Master Mix (toyobo).

### T302A-Introducing Primer Set

5'-CATCGTTCCTCCGGCTGGCGCAAAGGG-3' (SEQ ID NO: 12)
5'-CCCTTTGCGCCAGCCGGAGGAACGATG-3' (SEQ ID NO: 13)

The obtained polynucleotide fragment was treated with a restriction enzyme DpnI and then connected into a cyclic form using T4 Polynucleotide kinase (Takara Bio Inc.) and Ligation high Ver. 2 (TOYOBO Co., Ltd.), and the obtained cyclic polynucleotide was named a pRSF-HgPobA-T302A/Y393F plasmid (pRSF-HgPobA-TY plasmid).

### (3) Production of Protocatechuic Acid 5-Hydroxylases

The pRSF-HgPobA plasmid, the pRSF-HyPobA plasmid, the pRSF-PoPobA plasmid, the pRSF-HgPobA-TY plasmid and the pRSF-PaPobA plasmid described above were each introduced to *Escherichia coli* strain BL21 Star (DE3) (Merck KGaA), and *Escherichia coli* strains to which the target plasmids were introduced were selected by culturing on kanamycincontaining agar plates.

The obtained *Escherichia coli* strains were each added to a test tube containing 5 mL of liquid LB medium and cultured overnight at 37°C at 180 rpm, and thus preculture liquids were obtained.

LB liquid medium which was dispensed in a volume of 100 mL in baffled flasks having a total volume of 500 mL and sterilized was prepared, and kanamycin was added to a final concentration of 30 µg/L. The preculture liquids were added thereto to a final concentration of 1% and cultured with shaking at 37°C at 120 rpm.

When the bacterial cell turbidities (OD600) became 0.6, IPTG was added to a final concentration of 0.1 mM, and the strains were cultured for 12-18 hours while shaking at 20°C at 120 rpm to induce production of protocatechuic acid 5-hydroxylases.

### (4) Purification of Protocatechuic Acid 5-Hydroxylases

After the protein induction, the *Escherichia coli* strains were collected, suspended in a buffer (20 mM Tris-HCl buffer (pH 7.9)) and homogenized ultrasonically, and then the homogenates were centrifuged (8000 rpm, 10 minutes, 4°C) to collect the supernatants (cell-free extracts).

The cell-free extracts were subjected to a HisTrap HP column (GE healthcare) which was equilibrated with an equilibration buffer (20 mM Tris-HCl buffer (pH 7.9), 20 mM imidazole), and then fractions eluted with an eluent (20 mM Tris-HCl buffer (pH 7.9), 500 mM imidazole) were collected.

Then, by subjecting the eluted fractions to Amicon(R) Ultra Centrifugal Filters 0.5 mL 10k (Merck Milipore) and washing using a buffer, imidazole was removed from the protein solutions.

The protein concentrations of the purified enzyme liquids were measured using Bio-Rad Protein Assay Dye Reagent Concentrate (Bio-Rad, USA), and the purified enzyme liquids were diluted to 1 mg/mL with a buffer. Thus, purified enzyme liquids of protocatechuic acid 5-hydroxylases, HgPobA, HyPobA, PoPobA, HgPobA-TY and PaPobA, were prepared.

### (5) Examination of Identities of Amino Acid Sequences of Protocatechuic Acid 5-Hydroxylases and Existing PobA

Using the protocatechuic acid 5-hydroxylases related to HgPobA (SEQ ID NO: 20), HyPobA (SEQ ID NO: 21) and PoPobA (SEQ ID NO: 22) described above, the identities with the amino acid sequences of p-hydroxybenzoate hydroxylases (PobA) of the genus *Pseudomonas* and the genus *Corynebacterium* were examined.

The results are shown in Table 1 below. It can be seen that the identities of the protocatechuic acid 5-hydroxylases derived from the microorganisms according to the present technology were 60% or less with the amino acid sequences of p-hydroxybenzoate hydroxylases of the genus *Pseudomonas* and the genus *Corynebacterium.*

**[Table 1]**

| | *Pseudomonas aeruginosa* | *Corynebacterium glutamicum* | *Corynebacterium ammonigenes* | *Corynebacterium callunae* | *Corynebacterium efficiens* |
|---|---|---|---|---|---|
| *Hylemonella gracilis* | 59 | 37 | 40 | 37 | 38 |
| *Hydrogenephaga* sp. | 45 | 43 | 43 | 43 | 43 |
| *Polaromonas* sp. | 44 | 39 | 44 | 40 | 42 |

The identities of the amino acid sequences of the protocatechuic acid 5-hydroxylases according to the present technology and existing PobA
Amino acid sequence information of *Pseudomonas aeruginosa*-derived PobA (PaPobA) (SEQ ID NO: 28)
Amino acid sequence information of *Corynebacterium glutamicum*-derived PobA (CgPobA) (SEQ ID NO: 29)
Amino acid sequence information of *Corynebacterium ammoniagenes*-derived PobA (SEQ ID NO: 30)
Amino acid sequence information of *Corynebacterium* callunae-derived PobA (SEQ ID NO: 31)
Amino acid sequence information of *Corynebacterium* efficiens-derived PobA (SEQ ID NO: 32)

### (6) Measurement of Enzymatic Activities of Protocatechuic Acid 5-Hydroxylases

### <1> Reaction Kinetic Analysis

Using the purified enzyme liquids of HgPobA, HgPobA-TY, PoPobA, HyPobA and PaPobA described above, a reaction kinetic analysis was conducted.

For measuring the enzymatic activities, 100 µL of reaction liquids containing 20 mM Tris-HCl buffer (pH 7.9), 10 µM FAD, 200 µM NADPH, 1 mM pHBA or 2 mM PCA and 1 µg of the respective purified enzyme liquids were used.

The reaction was initiated by adding pHBA or PCA, and the absorbances of 340 nm were measured at 25°C for five minutes using a spectrophotometer U3900 (HITACHI).

The results are shown in Table 2. It can be seen that, when PCA was the substrate, HgPobA, PoPobA and HyPobA exhibited higher k_{cat} values than that of PaPobA and were effective for efficient production of gallic acid from PCA.

Moreover, the k_{cat} value for PCA of HgPobA-TY, in which two types of mutation were introduced in HgPobA, was 3.6 times larger than that of PaPobA, and the Kₘ value was also one third or less. It was found that, by introducing mutations, the excellent property as a PCA hydroxylase for hydroxylating PCA can be further improved.

Because the k_{cat} values for pHBA of PoPobA and HyPobA were one tenth to one twentieth of that of PaPobA and because the k_{cat} values for PCA were six to 12 times larger than that of PaPobA, it can be expected that the production reaction of gallic acid from PCA can be advanced dominantly over the production reaction of PCA from pHBA and that the yield of gallic acid in the reaction liquid is improved.

**[Table 2]**

| Enzyme | *p*HBA | | | PCA | | |
|---|---|---|---|---|---|---|
| | *k*_{cat}^{app} (*s*⁻¹) | *K*ₘ^{app} (mM) | *k*_{cat}^{app}/*K*ₘ^{app} (mM⁻¹*s*⁻¹) | *k*_{cat}^{app} (*s*⁻¹) | *K*ₘ^{app} (mM) | *k*_{cat}^{app}/*K*ₘ^{app} (mM⁻¹*s*⁻¹) |
| HgPobA | 5.6 | 0.035 | 160 | 0.74 | 0.26 | 2.8 |
| HgPobA-TY | 3.1 | 0.034 | 91 | 1.8 | 0.041 | 44 |
| PoPobA | 0.17 | 0.34 | 0.50 | 2.7 | 0.92 | 2.9 |
| HyPobA | 0.43 | 0.056 | 7.7 | 6.6 | 0.57 | 12 |
| PaPobA | 4.7 | 0.024 | 200 | 0.50 | 0.14 | 3.6 |

### Reaction kinetic analysis of novel protocatechuic acid 5-hydrhydroxylases

### <2> Analysis of Gallic Acid-Producing Activities

Using the purified enzyme liquids of HyPobA, PoPobA, HgPobA-TY and PaPobA described above, the gallic acid-producing activities were analyzed.

By adding 10 µM FAD, 0.5 mM NADPH, 2 mM PCA and 10 µg of the respective purified enzyme liquids to 20 mM Tris-HCl buffer (pH 7.9), 100 µL of reaction liquids were prepared. After reacting the reaction liquids at 30°C for 30 minutes, the concentrations of produced gallic acid were measured using HPLC (1200 Infinity Series: Hewlett Packerd).

As the results thereof, FIG. 2 shows the relative values of the activities of the enzymes of producing gallic acid from PCA, where the activity of PaPobA of producing gallic acid from PCA is regarded as 1.

It was found that, compared to the case using PaPobA, which is a known enzyme that is known to have high PCA-hydroxylating activity, HgPobA-TY can produce eight times more gallic acid, and HyPobA and PoPobA can produce 20 times more gallic acid.

### Example 2: Production of Gallic Acid through Fermentation Using Microbial Strains

### (1) Preparation of Corynebacterium Strains to Which Genes Encoding Protocatechuic Acid 5-Hydroxylases were Introduced

Genes encoding HyPobA, PoPobA, HgPobA-TY and CgPobA were introduced by the following procedures to strain CT07 which was genetically modified by the following procedures based on *Corynebacterium glutamicum* ATCC13032, which is known as a strain producing PCA from glucose.

### <1> Construction of Strain CT07

The promoters of tkt (cg1774), ppsA (cg0644), aroF^{fbr} (cg1129), aroG (cg2391) and qsuABCD operon (cg0501, cg0502, cg0503 and cg0504) of *Corynebacterium glutamicum* ATCC13032 (American Type Culture Collection) were replaced with the promoter of elongation factor Tu (cg0587) gene having structural and potent gene expression capability by the method described below.

DNAs including the gene promoter regions in the genes of *Corynebacterium glutamicum* ATCC13032 were synthesized, and the promoters were replaced with the promoter of elongation factor Tu.

The base sequence of the region cg1773 to cg1774 registered in a known database, such as NCBI (National Center for Biotechnology Information) and KEGG (Kyoto Encyclopedia of Genes and Genomes), was used for tkt (cg1774). Similarly, the base sequence of a region including cg0643 to cg0644 to cg0645 was used for ppsA(cg0644); the base sequence of a region including cg1127 to cg1128 to cg1129 was used for aroF^{fbr} (cg1129); the base sequence of a region including cg2393 to cg2392 to cg2391 was used for aroG (cg2391); and the base sequence of a region including cg0500 to cg0501 was used for qsuABCD operon (cg0501, cg0502, cg0503 and cg0504).

DNAs having sequences in which the promoter regions of the genes above were replaced with the promoter of elongation factor Tu gene and having the recognition sequence for SbfI at both ends of the DNAs were synthesized. The obtained DNAs were digested with SbfI and inserted to the SbfI site of pHG0 shown below, and a plasmid for homologous recombination was thus constructed. The plasmid was introduced to *Corynebacterium glutamicum* ATCC13032, and a strain in which the promoter of each gene was replaced with the promoter of elongation factor Tu was thus constructed.

Furthermore, mutation of P155L was introduced during the gene synthesis of aroF gene, and thus aroF^{fbr} was constructed. Moreover, the base sequence of cg2629 to cg2630 to cg2631 to cg2633 region including protocatechuic acid degradation gene pcaHG (cg2631 and cg2630) was obtained from the known database. A base sequence which lacked the pcaHG (cg2631 and cg2630) site alone from the base sequence was designed, and a gene in which the SbfI sequence was added to both ends of the base sequence was synthesized. The synthesized gene was digested with SbfI and inserted to the SbfI site of pHG0 constructed below, and a plasmid for homologous recombination was thus constructed. By introducing the plasmid to the above *Corynebacterium glutamicum* ATCC13032 strain to which the promoter of elongation factor Tu was introduced and causing homologous recombination with pcaHG gene on the chromosome, pcaHG gene on the chromosome of the *Corynebacterium glutamicum* ATCC13032 strain was deleted. The strain obtained in the above manner was named strain CT07.

### <2> Construction of Genome-Editing Vector

*Bacillus subtilis*-derived Levansucrase gene (sacB) which was artificially synthesized by Eurofins Genomics Inc. was introduced to the FspI site of a commercial vector, pHSG298 (Takara Bio Inc.), and vector pHG0 producing a lethal compound for microorganisms in a sucrose medium was constructed.

To introduce a target gene to strain CT07 by homologous recombination, a polynucleotide fragment of cg0658 (SEQ ID NO: 33) prepared by PCR and the *Corynebacterium glutamicum* ATCC13032 genome using a set of the primer below to which the SacI sequence was added and a primer to which the XbaI sequence was added was treated with restriction enzymes SacI and XbaI. Then, the polynucleotide fragment of cg0658 was introduced to the SacI and XbaI sites of vector pHG0 which was treated with restriction enzymes SacI and XbaI. The vector was named pHG0_0658.

SacI sequence-introducing cg0658 primer
   5'-GAGCTCATGCGTTTTGATCTCCATTC-3' (SEQ ID NO: 34)
XbaI sequence-introducing cg0658 primer
   5'-GAGCTCCTCTAGATTAGTTAGGGACCGTATGCG-3' (SEQ ID NO: 35)

### <3> Construction of Enzyme Expression Units

To express HyPobA, PoPobA, HgPobA-TY and CgPobA genes in *Corynebacterium glutamicum,* a kanamycin-resistant gene promoter (SEQ ID NO: 36) derived from pHSG298, which is a known promoter, was used.

Polynucleotide fragments having sequences in which the kanamycin-resistant gene promoter (SEQ ID NO: 36) was linked to HypobA (SEQ ID NO: 4), PopobA (SEQ ID NO: 7), HgPobA_TY (SEQ ID NO: 37) or CgPobA (SEQ ID NO: 17) were each obtained by artificial gene synthesis. Four polynucleotide fragments were produced using the obtained four polynucleotide fragments as templates by PCR using primer sets in which the XbaI sequence was added to the 5' end and the SalI sequence was added to the 3' end. The combinations of the template and the primers here are shown below.

A polynucleotide fragment was obtained using a polynucleotide fragment including HyPobA linked to the kanamycin-resistant gene promoter as a template by amplification by PCR using a XbaI sequence-introducing kanamycin-resistant gene promoter amplification Fw primer (SEQ ID NO: 38) and a HyPobASalI sequence-introducing Rv primer (SEQ ID NO: 39).

XbaI sequence-introducing kanamycin-resistant gene promoter amplification Fw primer
   5'-GAGCTCATGCGCACCCAGGTTGGTATC-3' (SEQ ID NO: 38)
HyPobASalI sequence-introducing Rv primer
   5'-GTCGACTTAGTCGATGGGCAAGCCCG-3' (SEQ ID NO: 39)

A polynucleotide fragment was obtained using a polynucleotide fragment including PoPobA linked to the kanamycin-resistant gene promoter as a template by amplification by PCR using the XbaI sequence-introducing kanamycin-resistant gene promoter amplification Fw primer (SEQ ID NO: 38) and a PoPobASalI sequence-introducing Rv primer (SEQ ID NO: 40).
PoPobASalI sequence-introducing Rv primer
5'-GTCGACTTATTCAATCGGAAGTCCAG-3' (SEQ ID NO: 40)

A polynucleotide fragment was obtained using a polynucleotide fragment including HgPobA-TY linked to the kanamycin-resistant gene promoter as a template by amplification by PCR using the XbaI sequence-introducing kanamycin-resistant gene promoter amplification Fw primer (SEQ ID NO: 38) and a HgPobA_SalI sequence-introducing Rv primer (SEQ ID NO: 41).
HgPobA_SalI sequence-introducing Rv primer
5'-GTCGACTTAGGCACCGAAGTCGAGGG-3' (SEQ ID NO: 41)

A polynucleotide fragment was obtained using a polynucleotide fragment including CgPobA linked to the kanamycin-resistant gene promoter as a template by amplification by PCR using the XbaI sequence-introducing kanamycin-resistant gene promoter amplification Fw primer (SEQ ID NO: 38) and a CgPobA_SalI sequence-introducing Rv primer (SEQ ID NO: 42).
CgPobA_SalI sequence-introducing Rv primer
5'-GTCGACTTACACTTCAAACCGCGGG-3' (SEQ ID NO: 42)

The four polynucleotide fragments obtained by PCR were digested using XbaI and SalI, and the digestion products were collected as digested DNA fragments related to protocatechuic acid 5-hydroxylases with a DNA collection column (Gel/PCR extraction kit manufactured by NIPPON Genetics Co., Ltd).

Similarly, the genome-editing vector pHG0_0658 was digested with XbaI and SalI, and digested pHG0_0658 was collected with a DNA collection column (Gel/PCR extraction kit manufactured by NIPPON Genetics Co., Ltd). Digested pHG0_0658 and the digested DNA fragments related to the protocatechuic acid 5-hydroxylases described above were ligated using Gibson Assembly Master Mix (New England Biolabs), and the four vectors shown below to which the enzyme expression units were introduced were obtained.
pHG0_0658_Pkm_HyPobA
pHG0_0658_Pkm_PoPobA
pHG0_0658_Pkm_HgPobA-TY
pHG0_0658_Pkm_CgPobA

### <4> Introduction of Enzyme Expression Units to Genome

The four plasmids produced above were each introduced to *Corynebacterium glutamicum* strain CT07 using the electroporation method, and the target genes were introduced by homologous recombination using cg0658 gene as the homologous recombination region. Microbial strains in which the plasmids were introduced to the genome were obtained by culturing in LB medium containing 20 µg/L kanamycin. The strains are gene-introduced microbial strains of *Corynebacterium glutamicum* strain CT07 having only one copy of a PobA gene per chromosome. The strains were named strain CG07-HY, strain CG07-PO, strain CG07-HG and strain CG07-CG.

### <5> Culture Test

Strain CG07-HY, strain CG07-PO, strain CG07-HG and strain CG07-CG were cultured with shaking at 30°C overnight in LB liquid medium containing 20 µg/L kanamycin, and thus preculture liquids were obtained. Moreover, strain CT07 without the introduction of the plasmids was cultured with shaking at 30°C overnight in antibiotics-free LB liquid medium, and thus a preculture liquid was obtained.

The preculture liquids were seeded at 1% in CGXII medium (pH 7.0) containing 5% glucose and cultured with shaking at 30°C for 72 hours. The culture liquids were appropriately diluted, and the concentrations of produced protocatechuic acid and gallic acid were measured using HPLC (1200 infinity series: Hewlett Packerd).

The results of the measurement of the concentrations of protocatechuic acid and gallic acid in the culture liquids of strain CG07-HY, strain CG07-PO, strain CG07-HG, strain CG07-CG and strain CT07 are shown in Table 3 below.

Although strain CT07 produced 7.3 g/L of protocatechuic acid, because PobA was not expressed, gallic acid (GA) was not produced. On the other hand, strain CG07-CG expressing CgPobA produced 7.5 g/L of protocatechuic acid and 0.0001 g/L of gallic acid. Moreover, CG07-HG expressing HgPobA-TY produced 5.5 g/L of protocatechuic acid and 0.65 g/L of gallic acid. Strain CG07-HY having HyPobA produced 7.3 g/L of protocatechuic acid and 0.03 g/L of gallic acid. Strain CG07-PO having PoPobA produced 7.3 g/L of protocatechuic acid and 0.0004 g/L of gallic acid.

The production amounts of gallic acid, based on the amount of CG07-CG having a known enzyme, CgPobA, regarded as 1, are as in the table below. Using HgPobA-TY, gallic acid (GA) in an amount 6500 times higher than the conventional amount could be produced.

**[Table 3]**

| Strain Name | PCA (g/L) | GA (g/L) | GA (relative value) |
|---|---|---|---|
| Strain CT07 | 7.3 | 0.00 | 0 |
| Strain CG07-CG | 7.5 | 0.0001 | 1 |
| Strain CG07-HG | 5.5 | 0.65 | 6500 |
| Strain CG07-HY | 7.3 | 0.03 | 300 |
| Strain CG07-PO | 7.2 | 0.0004 | 4 |

Of the natural-type enzymes which have been developed so far, the enzyme which achieves the maximum fermentation production of gallic acid is CgPobA or PaPobA, and it is reported that the production amounts of gallic acid using the enzymes are equivalent. When the protocatechuic acid 5-hydroxylase derived from the microorganism of the family *Comamonadaceae* according to the present invention is used, the yield of gallic acid can be expected to be increased largely compared to that using CgPobA. Moreover, by modifying the sequence of the protocatechuic acid 5-hydroxylase according to the invention, the yield of gallic acid in the fermented liquid can be further improved.

Furthermore, because gallic acid can be produced using a medium containing glucose as a substrate, a decrease in the production cost of gallic acid can be expected.

In Example 2, when HgPobA-TY was used, gallic acid could be produced in an amount 6500 times higher than that of the technology using strain CG07-CG, which was obtained by introducing CgPobA. This suggests that the known mutations which are known to enhance the ability of producing gallic acid when the mutations are introduced to PobA are also effective in HgPobA.

### [Low-pH Culture Test]

Strain CG07-HY, strain CG07-PO and strain CG07-HG were cultured with shaking at 30°C overnight in LB liquid medium containing 20 µg/L kanamycin, and thus preculture liquids were prepared. The preculture liquids were seeded at 1% in CGXII medium (pH 4.0) containing 5% glucose and cultured with shaking at 30°C for 72 hours. The culture liquids were appropriately diluted, and the concentrations of produced protocatechuic acid and gallic acid were measured using HPLC (1200 Infinity Series: Hewlett Packerd). Thus, it was observed that strain CG07-HY, strain CG07-PO and strain CG07-HG produced gallic acid also under the culture condition of pH 4.0. Under the culture condition, the used amount of the acid such as sulfuric acid and hydrochloric acid is decreased, and the risk of contamination of a strain other than the microbial strain of the present technology can be reduced.

### [High-Temperature Culture Test]

Strain CG07-HY, strain CG07-PO and strain CG07-HG were cultured with shaking at 30°C overnight in LB liquid medium containing 20 µg/L kanamycin, and thus preculture liquids were prepared. The preculture liquids were seeded at 1% in CGXII medium (pH 7.0) containing 5% glucose and cultured with shaking at 40°C for 72 hours. The culture liquids were appropriately diluted, and the concentrations of produced protocatechuic acid and gallic acid were measured using HPLC (1200 Infinity Series: Hewlett Packerd). Thus, it was observed that strain CG07-HY, strain CG07-PO and strain CG07-HG produced gallic acid also under the culture condition of 40°C. Under the culture condition, the culture efficiency is increased, and improvement of the production efficiency of gallic acid can be expected.

### Example 3: Expanded Production of Gallic Acid

Strain CG07-HG expressing HgPobA-TY was cultured with shaking at 30°C overnight in LB liquid medium containing 20 µg/L kanamycin, and thus a preculture liquid was obtained.

CGXII medium containing 5% glucose was prepared in a fermentation jar (manufactured by Marubishi Bioengineering Co., Ltd., product name MDL-8C), and the preculture liquid was seeded and cultured under the condition at 30°C for 56 hours at pH 6.8. As a result, 0.7 g/L of protocatechuic acid and 15.1 g/L of gallic acid were produced at the 56th hour of culturing. Determining from the production level, the developed fermentation production system can be used for actual production. Furthermore, by optimizing the culture condition of the fermentation production system, it can be expected that the production amount of gallic acid is further improved.

After heat sterilizing the culture liquid, bacterial cells were removed by centrifugation, filtering or the like. The culture filtrate was adjusted to pH 4.0 or less using sulfuric acid or the like. The insoluble protein and the like which generated in the process were removed by centrifugation, and by cooling the obtained liquid while appropriately stirring, crystals of gallic acid were obtained. The crystals were collected by centrifugation.

## Claims

1. A microbial strain obtained by introducing a gene encoding a protocatechuic acid 5-hydroxylase derived from a microorganism of the family *Comamonadaceae* or a protein having an identity of 70% or more with the amino acid sequence of the protocatechuic acid 5-hydroxylase.

2. The microbial strain according to claim 1, wherein the microorganism of the family *Comamonadaceae* is a microorganism selected from the genus *Hylemoenella,* the genus *Polaromonas* and the genus *Hydrogenophaga.*

3. The microbial strain according to claim 1, wherein the microbial strain is a microbial strain selected from the genus *Escherichia,* the genus *Rhodococcus,* the genus *Acinetobacter,* the genus *Bradyrhizobium,* the genus *Corynebacterium,* the genus *Pseudomonas,* the genus *Rhodopseudomonas,* the genus *Sinorhizobium,* the genus *Brevibacterium,* the genus *Novosphingobium* or the genus *Ralstonia.*

4. A method for producing gallic acid from protocatechuic acid through fermentation using the microbial strain according to claim 1.

5. A method for producing gallic acid through fermentation using the microbial strain according to claim 1 in a medium containing glucose.

6. The method for producing gallic acid according to claim 4 or 5, wherein the fermentation is conducted using a medium having pH 6.0 or less.

7. The method for producing gallic acid according to claim 4 or 5, wherein the fermentation is conducted under a temperature condition of 15°C to 45°C.

8. A method for producing gallic acid from protocatechuic acid using a protocatechuic acid 5-hydroxylase derived from a microorganism of the family *Comamonadaceae* or a protein having an identity of 70% or more with the amino acid sequence of the protocatechuic acid 5-hydroxylase.

9. A protein having an amino acid sequence in which a leucine at positions 199 to 209 has been replaced with a valine or a glycine in a protocatechuic acid 5-hydroxylase derived from a microorganism of the family *Comamonadaceae* or a protein having an identity of 70% or more with the amino acid sequence of the protocatechuic acid 5-hydroxylase.

10. A protein having an amino acid sequence in which a threonine at positions 294 to 304 has been replaced with an alanine in a protocatechuic acid 5-hydroxylase derived from a microorganism of the family *Comamonadaceae* or a protein having an identity of 70% or more with the amino acid sequence of the protocatechuic acid 5-hydroxylase.

11. The protein according to claim 9 or 10, wherein a tyrosine at positions 385 to 395 has been further replaced with a phenylalanine, a valine or an alanine.
